(19) Europäisches Patentamt
European Patent Office
Office européen des brevets

(11) Veröffentlichungsnummer: **0 112 587**
**B1**

(12) **EUROPÄISCHE PATENTSCHRIFT**

(45) Veröffentlichungstag der Patentschrift: **19.09.90**

(21) Anmeldenummer: **83113174.3**

(22) Anmeldetag: **28.12.83**

(51) Int. Cl.[5]: **C 07 C 39/12**, C 07 C 37/20, A 61 K 31/05, C 07 C 39/367, A 61 K 31/055

(54) **4-(1-(Phenyl)-1-(hydroxy)-prop-1-yl)-resorcinderivate, Verfahren zu ihrer Herstellung und diese Verbindungen enthaltende Arzneimittel.**

(30) Priorität: **28.12.82 HU 418982**

(43) Veröffentlichungstag der Anmeldung:
**04.07.84 Patentblatt 84/27**

(45) Bekanntmachung des Hinweises auf die Patenterteilung:
**19.09.90 Patentblatt 90/38**

(84) Benannte Vertragsstaaten:
**AT BE CH DE FR GB IT LI LU NL SE**

(56) Entgegenhaltungen:
**DE-A-2 260 138**

**CHEMICAL ABSTRACTS, Band 96, Nr. 5, 1. Februar 1982, Seite 16, Nr. 28204a, Columbus, Ohio, US; SZINAI I. et al.: "Metabolism of 4-(2'-diethylamino)ethoxy-alpha-ethylbenzhydrol (RGH 6201) in rat urine and bile" & DRUGS, BIOCHEM. METAB., SCI. MATER. PAP COLLOQ. 1981, 239-42**

(73) Patentinhaber: **RICHTER GEDEON VEGYESZETI GYAR R.T.**
**Gyömröi ut 19-21**
**H-1475 Budapest X (HU)**

(72) Erfinder: **Tóth, Edit, Dipl.-Ing.**
**Szabolcska u. 7**
**H-1114 Budapest (HU)**
Erfinder: **Törley, József, Dipl.-Ing.**
**Katona J. u. 41**
**H-1137 Budapest (HU)**
Erfinder: **Fekete, György, Dr.**
**Széher u. 62**
**H-1021 Budapest (HU)**
Erfinder: **Szporny, László, Dr.**
**Szabolcska u. 7**
**H-1114 Budapest (HU)**
Erfinder: **Vereczkey, László, Dr.**
**Lajos u. 109**
**H-1036 Budapest (HU)**
Erfinder: **Pálosi, Eva, Dr.**
**Vend u. 21**
**H-1025 Budapest (HU)**
Erfinder: **Klebovich, Imre, Dr.**
**Karvaly u. 4**
**H-1125 Budapest (HU)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 112 587 B1

Courier Press, Leamington Spa, England.

(72) Erfinder: **Vittay, Pál, Dr.**
**József krt. 14**
**H-1085 Budapest (HU)**
Erfinder: **Görög, Sándor, Dr. Dipl.-Chem.**
**Vajda P. u. 43**
**H-1089 Budapest (HU)**
Erfinder: **Hajdu, István, Dipl.-Chem.**
**Tátra tér B/4**
**H-1205 Budapest (HU)**

(74) Vertreter: **Beszédes, Stephan G., Dr.**
**Patentanwalt**
**Münchener Strasse 80a Postfach 1168**
**D-8060 Dachau (DE)**

**Beschreibung**

Die Erfindung betrifft neue 4-[1-(Phenyl)-1-(hydroxy)-prop-1-yl]-resorcinderivate, ein Verfahren zu ihrer Herstellung und diese Verbindungen enthaltende Arzneimittel.

Gegenstand der Erfindung sind 4-[1-(Phenyl)-1-(hydroxy)-prop-1-yl]-resorcinderivate der allgemeinen Formel

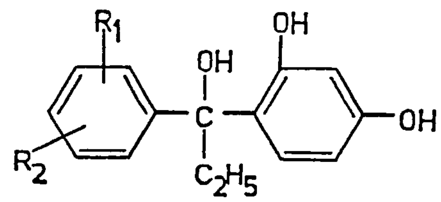

I ,

worin $R_1$ und $R_2$, die gleich oder verschieden sein können, für Wasserstoffatome, Halogenatome, Trihalogenmethylreste beziehungsweise Alkyl- beziehungsweise Alkoxyreste mit je 1 bis 3 Kohlenstoffatom(en) stehen.

Die Herstellung von Verbindungen ähnlicher Struktur ist in den folgenden Literaturstellen beschrieben: C.A. *22,* 410[1]; *35,* 1781[2]; *40,* 4712[5]; *42,* P 1015 g; *47,* 9548 e; *50,* 12390 c; *50,* 2509 i; *55,* 17915 e; *55,* 15413 b; *75,* P 103682 b; *76,* P 119921 k; *82,* 16477 q; *90,* 86082 q; *92,* 52927 b, es wird jedoch nirgends erwähnt, daß die hergestellten Verbindungen eine pharmazeutische Wirkung haben.

Vorzugsweise ist beziehungsweise sind das beziehungsweise die Halogenatom(e), für das beziehungsweise die $R_1$ und/oder $R_2$ stehen kann beziehungsweise können, [ein] Chlor-, Fluor- und/oder Bromatom(e), insbesondere [ein] Chlor- und/oder Fluoratom(e).

Es ist auch bevorzugt, daß der beziehungsweise die Trihalogenmethylrest(e), für den beziehungsweise die $R_1$ und/oder $R_2$ stehen kann beziehungsweise können, [ein] Trifluormethylrest(e) und/oder Trichlormethylrest(e), insbesondere der beziehungsweise die erstere(n), ist beziehungsweise sind.

Ferner ist es bevorzugt, daß der beziehungsweise die Alkyl- und/oder Alkoxyrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

Besonders bevorzugte erfindungsgemäße 4-[1-(Phenyl)-1-(hydroxy)-prop-1-yl]-resorcinderivate sind
4-[1-(3-Trifluormethyl-phenyl)-1-hydroxy-propyl]-resorcin,
4-[1-(2-Trifluormethyl-phenyl)-1-hydroxy-propyl]-resorcin,
4-[1-(4-Fluorphenyl)-1-hydroxy-propyl]-resorcin,
4-[1-(2-Methoxyphenyl)-1-hydroxy-propyl]-resorcin,
4-[1-(4-Chlorphenyl)-1-hydroxy-propyl]-resorcin,
4-[1-(4-Trifluormethyl-phenyl)-1-hydroxy-propyl]-resorcin,
4-[1-(3-Chlorphenyl)-1-hydroxy-propyl]-resorcin und
4-[1-(2,5-Dimethylphenyl)-1-hydroxy-propyl]-resorcin.

Gegenstand der Erfindung ist auch ein Verfahren zur Herstellung der erfindungsgemäßen Verbindungen, welches dadurch gekennzeichnet ist, daß in an sich bekannter Weise

a) 2,4-Dihydroxypropiophenon mit eine Phenylgruppe aufweisenden Grignard-Verbindungen der allgemeinen Formel

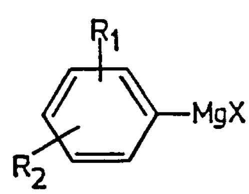

II ,

worin

$R_1$ und $R_2$ wie oben festgelegt sind und

X für ein Halogenatom steht, umgesetzt werden oder

b) 2,4-Dihydroxypropiophenon mit eine Phenylgruppe aufweisenden metallorganischen Verbindungen der allgemeinen Formel

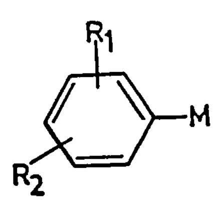

III ,

worin

$R_1$ und $R_2$ wie oben festgelegt sind und

M für ein Alkalimetallatom, vorzugsweise Lithium-, Natriu- oder Kaliumatom, steht umgesetzt werden oder

c) 2,4-Dihydroxybenzophenone der allgemeinen Formel

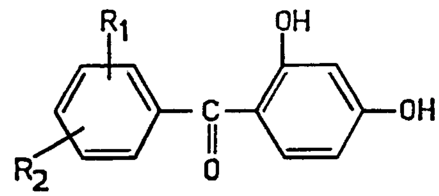

IV ,

worin $R_1$ und $R_2$ wie oben festgelegt sind, mit eine Äthylgruppe aufweisenden metallorganischen Verbindungen, insbesondere Äthylmagnesiumhalogeniden oder Äthyllithium, umgesetzt werden.

Die erfindungsgemäßen 4-[1-(Phenyl)-1-(hydroxy)-prop-1-yl]-resorcinderivate können auch durch andere bekannte Verfahren hergestellt werden.

In "Chemical Abstracts", Band 96, Nr. 5, 1. Februar 1982, Seite 16, Nr. 28204a wird der Stoffwechsel von 4-(2'-Diethylamino)-ethoxy-α-ethylbenzhydrol in Rattenversuchen beschrieben. Diese Benzhydrole sind von den erfindungsgemäßen Resorcinderivaten strukturell sehr verschieden und man findet auch in dieser Literaturstelle keinen Hinweis auf eine Wirkung gegenüber einer Alkoholintoxikation, wie sie den erfindungsgemäßen Resorcinderivaten zukommt.

Die DE—OS 2 260 138 beschreibt Bisphenolalkane der dort angegebenen Formeln I und II, von denen einerseits die ersteren sich strukturell von den erfindungsgemäßen Resorcinderivaten durch das Fehlen der Hydroxygruppe in der Stellung 3 des einen Benzolrings und das Vorliegen von je 1 Hydroxygruppe an jedem Benzolring und die zweiten durch das Fehlen von zwei Hydroxygruppen in den Stellungen 3 und 4 des einen Benzolrings unterscheiden und andererseits alle bekannten bekannten Bisphenolalkane eine hypolipidämische und antihyperglycämische Wirksamkeit besitzen und damit auch bezüglich der Indikation von den erfindungsgemäßen Resorcinderivaten verschieden sind.

Die Ausgangsstoffe sind bekannt beziehungsweise können nach literaturbekannten Verfahren hergestellt werden. Die Ausgangsverbindungen der allgemeinen Formel (II) sind zum Beispiel erhältlich, indem man aus entsprechend substituierten Phenylhalogeniden in an sich bekannter Weise, zum Beispiel nach der Methode von M.S. Kharash u.a. (Grignard reactions of nonmetallic substances, Ed. Prentice-Hall Inc. /1954/, S. 5—90), das Grignard-Reagens bereitet.

Die metallorganischen Verbindungen der allgemeinen Formel (III) können zum Beispiel nach Houben-Weyl: Methoden der organischen Chemie, Bd. XIII/1, S. 134—159 (1970) hergestellt werden.

Die Dihydroxybenzophenone der allgemeinen Formel (IV) sind zum Beispiel durch die Friedel-Crafts-Ketonsynthese oder die Fries-Reaktion erhältlich (G.A. Olah: Friedel-Crafts and related reactions, Bd. III/1, Ed.: Interscience Publishers, S. 1—63 u. 499—511 /1964/).

Gemäß einer bevorzugten Ausführungsform der Variante a) des Verfahrens wird das 2,4-Dihydroxypropiophenon in einem wasserfreier organischen Lösungsmittel mit Arylmagnesiumhalogeniden, vorzugsweise Arylmagnesiumchloriden, der allgemeinen Formel (II) umgesetzt. Das Grignard-Reagens wird im mindestens Dreifachen der äquivalenten Menge eingesetzt. Die Umsetzung wird zweckmäßig in einem aprotischen Lösungsmittel vorgenommen, geeignet sind zum Beispiel: aliphatische Äther, wie Diäthyläther, Di-n-propyläther, Diäthylenglykoldimethyläther, alicyclische Äther, zum Beispiel Tetrahydrofuran und Dioxan, aliphatische oder aromatische Kohlenwasserstoffe, zum Beispiel Ligroin, Benzol, Toluol, Xylol oder Gemische der aufgeführten Lösungsmittel. Die Umsetzung wird bei Temperaturen zwischen −30°C und dem Siedepunkt des Lösungsmittels, vorzugsweise zwischen −10 und 100°C, vorgenommen.

Nach Ablauf der Reaktion wird der Grignard-Komplex mit verdünnten Mineralsäuren, zum Beispiel Essigsäure, vorzugsweise jedoch mit wäßriger Ammoniumchloridlösung zersetzt und die entstandene Verbindung der allgemeinen Formel (I) isoliert. Das Produkt kann zum Beispiel durch Umkristallisieren gereinigt werden.

Gemäß der Verfahrensvariante b) wird das 2,4-Dihydroxypropiophenon in Abwesenheit von Wasser mit den metallorganischen Verbindungen der allgemeinen Formel (III), vorzugsweise dem entsprechend substituierten Phenyllithium, in einem inerten organischen Lösungsmittel umgesetzt. Die Verbindung der allgemeinen Formel (III) wird im mindestens Dreifachen der äquimolaren Menge verwendet. Als Lösungsmittel werden aprotische Lösungsmittel eingesetzt, zum Beispiel: Äther, wie Tetrahydrofuran oder Dioxan, ferner Benzol, Toluol, Xylol, Hexan, Dimethylsulfoxyd, Hexamethylphosphoramid oder Gemische der aufgeführten Lösungsmittel. Die Reaktionstemperatur kann innerhalb weiter Grenzen variieren und liegt zwischen −60 und 100°C, vorzugsweise −40 und 80°C. Das Reaktionsgemisch wird auf die unter a) bereits beschriebene Weise aufgearbeitet. Das Produkt kann zum Beispiel chromatographisch und/oder durch Umkristallisieren gereinigt werden.

Zur Durchführung der Verfahrensvariante c) werden die Benzophenone der allgemeinen Formel (IV) in einem wasserfreien organischen Lösungsmittel mit vorzugsweise Äthylmagnesiumbromid oder Äthylmagnesiumjodid oder Äthyllithium umgesetzt. Dabei wird die äthylgruppenhaltige metallorganische Verbindung im wenigstens Dreifachen der äquimolaren Menge eingesetzt. Als Lösungsmittel kommen bevorzugt Äther, aliphatische und aromatische Kohlenwasserstoffe oder Gemische dieser Lösungsmittel in Betracht. Die Umsetzung wird bei Temperaturen zwischen −60°C und dem Siedepunkt des Lösungsmittels, vorzugsweise zwischen −40 und 100°C, vorgenommen.

Der metallorganische Komplex wird mit einer verdünnten anorganischen oder organischen Säure, vorzugsweise jedoch mit wäßriger Ammoniumchloridlösung zersetzt und die gebildete Verbindung der allgemeinen Formel (I) isoliert. Das Produkt kann zum Beispiel durch Behandeln mit einem Lösungsmittel, durch Auskochen, Verreiben, Umfällen, Chromatographie, Umkristallisieren oder Kombinationen dieser Methoden gereinigt werden.

Die Verfahrensvarianten a)—c) werden vorzugsweise unter Schutzgas, zum Beispiel Stickstoff oder Argon, ausgeführt.

Die Verbindungen der allgemeinen Formel (I) sind zur Behandlung akuter Äthanolintoxikation geeignet und für diesen Zweck ausgedehnt verwendbar. Die akute Alkoholintoxikation ist vor allem durch Euphorie, allgemeine Stimuliertheit, Ataxie, Somnolenz und paralytische Zustände charakterisiert. Die Gefahren dieses toxischen, krankhaften Zustandes sind bekannt und nicht zu vernachlässigen. Die intoxierte Person gefährdet ihre Umgebung (z.B. Trunkenheit am Steuer) und sich selbst.

Die akute Alkoholintoxikation ist für den ischämischen Gehirninfarkt ein bedeutender Risikofactor (Hillbom, M. u.a.: Lancet *2*, 1181 /1978/; Stroke *12*, 422 /1981/). Für den alkoholintoxierten Zustand gibt es kein geeignetes Antidotum. Die durch Alkohol ausgelöste lokomotorische Hyperaktivität wird an Mäusen durch α-Methyl-para-thyrosin in einem Dosisbereich normalisiert, in dem die Verbindung die spontane lokomotorische Aktivität der Tiere beeinträchtigt (Carlsson, A. u.a.: Psychopharm. *26*, 307 /1972/). Die narkotisierende Wirkung des Alkohols wird durch Stimulantien (Coffein, Amphetamin) verringert, die motorische Inkoordination (Ataxie) jedoch gleichzeitig prolongiert (Wallagsen, H. u.a.: Actions of alcohol, Amsterdam, Elsevier, 1970; Rech, R.H. u.a.: Ann. N.Y. Acad. Sci. *28*, 426, 1976; Todzy, I. u.a.: Psychopharm. *59*, 143, 1978). Die alkoholische Intoxikation, die Narkose wird durch L-Cystein verkürzt (Sprince, H. u.a.: Agents and Actions *4*, 125, 1974; Nagasawa, H.T. u.a.: Life Sci. *17*, 707, 1975). L-Cystein wurde zu den folgenden Versuchen über Schlaf unter Alkoholeinfluß als Referenzsubstanz verwendet.

Zur Messung der Änderung der Zeitspanne der durch Äthanol hervorgerufenen Narkose wurden 16 Stunden lang ausgehungerte, 160—180 g schwere Hann.-Wistar-Ratten beiderlei Geschlechts verwendet. Jede Gruppe bestand aus 10 Tieren, die mit entsprechenden Dosen der Versuchsverbindungen oral behandelt wurden. Eine Stunde nach der Behandlung wurde den Tieren in einer Dosis von 3,5 g/kg intraperitoneal Äthanol appliziert. Die Schlafdauer wurde vom Ausfall des Aufrichtreflexes (righting Reflexes) bis zur spontanen Korrektur der Körperhaltung gemessen. Für jede Gruppe wurde die durchschnittliche Schlafzeit, die Standard-abweichung und das Ergebnis in Prozent der Kontrolle berechnet. Die Kontrolle erhielt Placebo und ebenfalls 3,5 g/kg Alkohol. Die Ergebnisse sind in der folgenden Tabelle 1 angegeben, in der $\bar{x}$ ±S.E. Durchschnittswert ± Standardabweichung bedeutet.

Schlafdauer der Kontrolle: 99,6 ± 6,55 ($\bar{x}$ ±S.E.) min A = 4-[1-(3-Trifluormethyl-phenyl)-1-hydroxy-propyl]-resorcin

Aus den Daten der Tabelle ist ersichtlich, daß die Verbindung der allgemeinen Formel (I) die auf das Zentralnervensystem ausgeübte deprimierende Wirkung bedeutend vermindert und die Dauer der Narkose dosisabhängig wesentlich verkürzt. Zum Erreichen der gleichen Wirkung ist von der Referenzsubstanz L-Cystein das 500fache erforderlich; in einer dem hundertsten Teil der Cystein-Dosis entsprechenden Dosis übertreffen die erfindungsgemäßen Verbindungen die Wirkung des L-Cysteins bei weitem.

TABELLE 1

| Behandlung | Dosis mg/kg | Schlafdauer (Kontrolle ±S.E.) | $LD_{50}$ mg/kg p.o. |
|---|---|---|---|
| A | 0,3 | 70 ± 11,4 | 1250,0 |
| | 1,0 | 60 ± 9,4 | |
| | 5,0 | 36 ± 4,0 | |
| | 20,0 | 30 ± 5,5 | |
| L-Cystein | 500,0 | 63 ± 4,2 | 1890 |
| Kontrolle | | 100 ± 6,6 | |

Infolge seiner stimulierenden Wirkung löst Alkohol neben der Deprimierung des Zentralnervensystems auch eine von der Dosis abhängige Hyperaktivität aus. Die von den erfindungsgemäßen Verbindungen auf die durch Alkohol ausgelöste Hyperaktivität ausgeübte Wirkung wurde an 16—18 g schweren BALB/c Mäusen beiderlei Geschlechts untersucht. Jede Gruppe bestand aus 15 Tieren. Die Testsubstanzen wurden in einer Dosis von 40 mg/kg oral verabreicht, eine Stunde vor der intraperitonealen Applikation von Placebo beziehungsweise 2 g/kg Äthanol. Die Kontrolle wurde mit Placebo behandelt. Die lokomotorische Aktivität der Tiere wurde mit dem Motimeter Animex BSE 2 Stunden lang gemessen. Die für die Gesamtzahl der Bewegungen gemessenen Werte sind in der folgenden Tabelle 2 in Prozent der Kontrolle ausgedrückt.

TABELLE 2

| Behandlung | Dosis (mg/kg) Verbindung | $C_2H_5OH$ | Lokomotorische Aktivität in % der Kontrolle |
|---|---|---|---|
| mit Placebo behandelte Kontrolle | — | — | 100 ± 8,8 |
| Äthanol + Placebo | — | 2000 | 180 ± 10,3 |
| A + Placebo | 40 | — | 110 ± 10,3 |
| A + Äthanol | 40 | 2000 | 98 ± 9,5 |

mit Placebo behandelte Kontrolle:
$\bar{x} \pm$ S.E. = 2941 ± 258,11 Bewegungen/2 Stunden.

Aus der Tabelle 2 ist ersichtlich, daß die erfindungsgemäße Verbindung die durch Äthanol ausgelöste Hyperaktivität normalisiert; die lokomotorische Aktivität der mit Testverbindung + Alkohol behandelten Tiere weicht nicht von der mit Placebo behandelten Kontrolle ab. Die spontane lokomotorische Aktivität der Tiere wird durch die erfindungsgemäßen Verbindung nicht verändert.

Die von den erfindungsgemäßen Verbindungen auf die durch Alkohol verursachte Ataxie und auf die Änderung des Muskeltonus ausgeübte Wirkung wurde am Drehstab untersucht. Trainierte und ausgewählte BALB/c-Mäuse beiderlei Geschlechts (Gewicht 16—18 g) wurden mit 40 mg/kg der Testverbindungen oral behandelt, eine Stunde vor der intraperitonealen Applikation von 2,5 g/kg Alkohol. Die Zeit, die die Tiere auf dem Drehstab zu verbleiben vermochten, wurde 60, 90 und 120 Minuten nach der Verabreichung des Alkohols gemessen. Die Tiere, deren Koordination intakt war, blieben 120 Sekunden auf dem Drehstab. Jede Behandlung wurde an einer aus 10 Tieren bestehenden Gruppe vorgenommen. Die Ergebnisse sind in der folgenden Tabelle 3 zusammengestellt.

TABELLE 3

| Behandlung | Dosis, mg/kg A | $C_2H_5OH$ | auf der Drehstange gebliebene Tiere (%) nach 60 | 90 | 120 min |
|---|---|---|---|---|---|
| Äthanol | | 2500 | 10 | 40 | 50 |
| A + Äthanol | 40 | 2500 | 40 | 60 | 100 |
| A | 40 | | 100 | 100 | 100 |

Aus der Tabelle 3 ist ersichtlich, daß die erfindungsgemäßen Verbindungen, an sich verabreicht, in einer von 40 mg/kg die motorische Koordination der Tiere nicht beeinflussen, gleichzeitig jedoch die durch Alkohol ausgelöste Ataxie bedeutend verringern und die auf das Zentralnervensystem ausgeübte deprimierende Wirkung des Alkohols kompensieren.

Durch Untersuchungen auf die im folgenden angegebene Weise wurde festgestellt, daß die Verbindungen keinerlei sonstige Wirkungen auf das Zentralnervensystem ausüben: Elektroschock (Swinyard, E.A., Brown, W.C., Goodman, L.S.: J. Pharmacol. Exp. Ther. *106*, 319 /1952/), Metrazolkrampf (Everett, G.M., Richards, R.K.: J. Pharmacol. Exp. Ther. *81*, 402 /1944/), Thiosemicarbazidkrampf (Da Vanzo,

J.P., Greig, M.E., Cormin, M.A.: Amer. J. Physiol. *201,* 833 /1961/), Strychninkrampf (Kerley, T.L., Richards, A.G., Begley, R.W., Abreu, B.B., Wesver, L.C.: J. Pharmacol. Exp. Ther. *132,* 360 /1961/), Nikotinkrampf (Stone, C.A., Mecklenburg, K.L., Torhans, M.L.: Arch. Int. Pharmacodyn. *117,* 419 /1958/), Drehstab (Kinnard, W.J., Carr, C.J.: J. Pharmacol. Exp. Ther. *121,* 354 /1957/), Physostigminletalität (Nose, T. und Kojima, M.: Europ. J. Pharmacol. *10,* 83 /1970/), yohimbinpotenzierende Wirkung (Quinton, R.M.; Brit. J. Pharmacol. *21,* 51 /1963/), schmerzstillende Wirkung (Bianchi, C., Franceschini, J.: Brit. J. Pharm. Chemother. *9,* 280 /1954/).

Die akute Toxizität der erfindungsgemäßen Verbindungen wurde an 160—180 g schweren männlichen Hann.-Wistar-Ratten bestimmt. Aus dem Prozentsatz der verendeten Tiere wurde mittels Probitanalyse berechnet, welche Dosis 50 %ige Letalität verursacht. Die Tiere wurden 14 Tage lang beobachtet. Die $LD_{50}$-Werte sind in der Tabelle 1 angegeben.

Die Ergebnisse zusammenfassend kann man sagen, daß die erfindungsgemäßen Verbindungen die durch Alkohol alterierten Betragensformen in günstiger Richtung beeinflussen. Die von Alkohol auf das Zentralnervensystem ausgeübte stimulierende und deprimierende Wirkung wird kompensiert, die Ausnüchterungszeit wird verkürzt; die Toxizität der Verbindungen ist niedrig, ihre therapeutische Breite groß.

Die erfindungsgemäßen Verbindungen können zu Arzneimittelpräparaten zubereitet werden. Die Präparate können oral, rektal und/oder parenteral verabreicht werden. Zur oralen Darreichung können Tabletten, Dragees oder Kapseln hergestellt werden. Bei der Herstellung von oral verabreichbaren Formen werden als Streckmittel zum Beispiel Milchzucker oder Stärke verwendet. Als Binde- und Granuliermittel kommen zum Beispiel Gelatine, Carboxymethylcellulose-natrium, Methylcellulose, Polyvinylpyrrolidon oder Stärkekleister in Frage. Als Sprengmittel werden in erster Linie Kartoffelstärke oder mikrokristalline Cellulose zugesetzt, jedoch können beispielsweise auch Ultraamylopektin oder Formaldehydcasein verwendet werden. Als Antihaftmittel und Gleitmittel kommen z.B. Talk, kolloidale Kieselsäure, Stearin, Ca- und Mg-Stearat in Frage.

Die Tabletten können zum Beispiel durch Naßgranulieren und anschließendes Pressen hergestellt werden. Das Gemisch aus Wirkstoff und Füllstoffen sowie gegebenenfalls ein Teil des Sprengmittels werden mit der wäßrigen, alkoholischen oder wäßrig-alkoholischen Lösung der Bindemittel in einer geeigneten Vorrichtung granuliert, und das Granulat wird getrocknet. Zu dem trockenen Granulat werden der Rest des Sprengmittels, ferner die Antihaftmittel und das Gleitmittel gegeben, und das Gemisch wird zu Tabletten gepreßt. Gegebenenfalls können die Tabletten zur Erleichterung der Dosierung mit Teilungsmarkierungen versehen werden. Die Tabletten können auch unmittelbar durch Pressen eines Gemisches aus dem Wirkstoff und geeigneten Hilfsstoffen hergestellt werden.

Die Tabletten können gegebenenfalls unter Verwendung der in der Arzneimittelindustrie üblichen Schutz-, Geschmacks- und Farbstoffe, zum Beispiel Zucker, Cellulose-derivate, wie Methyl- oder Äthylcellulose oder Carboxymethylcellulose-natrium, Polyvinylpyrrolidon, Calciumphosphat, Calciumcarbonat, Lebensmittelfarbstoffe, Lebensmittelfarblacke, Aromastoffe und Eisenoxydpigmente, auf die übliche Weise dragiert werden.

Zur Herstellung von Kapseln wird das Gemisch aus Wirkstoff und Füllstoffen in Kapseln gefüllt.

Zur rektalen Anwendung werden Suppositorien hergestellt. Diese enthalten außer dem Wirkstoff eine Trägermasse, das sog. Adeps pro suppositori. Als solches kommen Pflanzenfette, zum Beispiel gehärtete Pflanzenöle, die Triglyceride von Fettsäuren mit 12—18 Kohlenstoffatomen, vorzugsweise die Trägerstoffe der Markenbezeichnung Witepsol®, in Betracht. Der Wirkstoff wird in der geschmolzenen Trägermasse homogen verteilt und das erhaltene Gemisch zu Suppositorien vergossen.

Zur parenteralen Verabreichung werden Präparate in Injektionslösungsform hergestellt. Dazu werden die Wirkstoffe, gegebenenfalls in Gegenwart von Lösungsvermittlern, wie Polyoxyäthylensorbitmonolaurat, -monooleat oder -monostearat (Tween 20®, Tween 60®, Tween 80®), in destilliertem Wasser und/oder unterschiedlichen organischen Lösungsmitteln, zum Beispiel Glykoläthern, gelöst. Die Injektionslösungen können ferner verschiedene Hilfsstoffe, zum Beispiel Konservierungsstoffe, wie Benzylalkohol, p-Oxybenzoesäuremethyl- oder -propylester, Benzalkoniumchlorid oder Phenylmercuriborat, zum Binden von Metallspuren Komplexbildner, Äthylendiamen-tetraessigsäure, Puffersubstanzen oder Stoffe zum Einstellen des pH-Wertes sowie gegebenenfalls Lokalanästhetika, wie Lidocain, enthalten. Die Injektionslösungen werden vor dem Abfüllen filtriert und, nachdem sie in Ampullen gefüllt wurden, sterilisiert.

Die Tagesdosis beträgt, abhängend vom Zustand des Kranken, 0,1—300 mg/kg, vorzugsweise 2,0—160 mg/kg, und wird zweckmäßig in kleineren Einzeldosen verabreicht.

Die Erfindung wird an Hand der folgenden Beispiele näher erläutert.

### Beispiel 1

4-[1-(3-Trifluormethyl-phenyl)-1-hydroxy-propyl]-resorcin

Zu dem aus 14,6 g Magnesiumspänen und 135 g 3-Trifluormethyl-brombenzol in 330 ml Tetrahydrofuran bereiteten Grignard-Reagens wird bei 50°C tropfenweise die Lösung von 16,6 g 2,4-Dihydroxypropiophenon in 83 ml Tetrahydrofuran gegeben und das Gemisch bei der angegebenen Temperatur 30 Minuten lang gerührt. Nach dem Abkühlen wird das Gemisch unter Rühren in kalte, 20 %ige wäßrige Ammoniumchloridlösung eingegossen. Die Phasen werden voneinander getrennt, und die

wäßrige Phase wird mit Tetrahydrofuran extrahiert. Die vereinigten organischen Phasen werden mit gesättigter wäßriger Kochsalzlösung gewaschen, über wasserfreiem Magnesiumsulfat getrocknet und im Vakuum von Lösungsmittel befreit. Der Rückstand wird in Methanol gelöst, die Lösung mit Aktivkohle geklärt und nach dem Filtrieren unter vermindertem Druck eingedampft. Notwendigenfalls wird das Produkt durch Kristallisieren aus einem Äthylacetat/n-Hexan-Gemisch gereinigt. Man erhält 26,6 g der Titelverbindung, die bei 137—138°C schmilzt.

Elementaranalyse für die Summenformel $C_{16}H_{15}F_3O_3$
berechnet, %: C 61,53 H 4,84 F 18,25
gefunden, %: C 61,71 H 4,97 F 18,51

Beispiel 2

4-[1-(2-Trifluormethyl-phenyl)-1-hydroxy-propyl]-resorcin

Zu dem aus 18,9 g n-Butyllithium und 65,2 g 2-(Trifluormethyl)-brombenzol in 480 ml wasserfreiem Äther hergestellten 2-Trifluormethylphenyllithium wird unter Rühren bei einer Temperatur zwischen 0 und −5°C tropfenweise die Lösung von 8,3 g 2,4-Dihydroxypropiophenon in 85 ml wasserfreiem Äther gegeben. Das Reaktionsgemisch wird bei Raumtemperatur 2 Stunden lang gerührt, dann abgekühlt und in eiskalte 10 %ige wäßrige Ammoniumchloridlösung gegossen. Die Phasen werden voneinander getrennt, und die wäßrige Phase wird mit Äther extrahiert. Die organische Phase wird mit Wasser neutral gewaschen, über wasserfreiem Magnesiumsulfat getrocknet, mit Aktivkohle geklärt, filtriert und das Lösungsmittel unter vermindertem Druck abdestilliert. Man erhält 15 g eines öligen Rohproduktes. Dieses wird mit Dichlormethan als Eluîermittel an einer Silikagelsäule chromatographiert. Das Dichlormethan wird unter vermindertem Druck abdestilliert und der feste Rückstand aus einem n-Hexan/Äthylacetat-Gemisch umkristallisiert. Man erhält 6,9 g der Titelverbindung, die bei 143—144°C schmilzt.

Elementaranalyse für die Summenformel $C_{16}H_{15}F_3O_3$
berechnet, %: C 61,53 H 4,84 F 18,25
gefunden, %: C 61,75 H 5,02 F 18,33.

Beispiel 3

4-[1-(4-Fluorphenyl)-1-hydroxy-propyl]-resorcin

Aus 4,8 g Magnesiumspänen und 31,2 g Äthyljodid wird in 80 ml wasserfreiem Äther Äthylmagnesiumjodid bereitet. Zu der gekühlten Lösung der Grignard-Verbindung wird tropfenweise die Lösung von 7,9 g 4-Fluor-2',4'-dihydroxy-benzophenon in 80 ml wasserfreiem Äther gegeben, wobei darauf zu achten ist, daß die Temperatur nicht über −5°C asteigt. Das Reaktionsgemisch wird 30 Minuten lang bei 0°C, dann eine Stunde lang unter Kochen am Rückfluß gerührt und nach dem Abkühlen in eine eiskalte Ammoniumchloridlösung gegossen. Die ätherische Phase wird abgetrennt und die wäßrige Phase mit Äther extrahiert. Die organischen Phasen werden vereinigt, mit Wasser neutral gewaschen, über wasserfreiem Magnesiumsulfat getrocknet und nach dem Filtrieren unter vermindertem Druck vom Lösungsmittel befreit. Der feste Rückstand wird aus einem n-Hexan/Äthylacetat-Gemisch kristallisiert. Man erhält 5,1 g der Titelverbindung, die bei 126°C schmilzt.

Elementaranalyse für die Summenformel $C_{15}H_{15}FO_3$
berechnet, %: C 68,69 H 5,76 F 7,24
gefunden, %: C 68,85 H 5,93 F 7,44.

Beispiel 4

4-[1-(2-Methoxyphenyl)-1-hydroxy-propyl]-resorcin

Zu der auf −10°C gekühlten Lösung von aus 29,4 g Äthylbromid und 3,7 g metallischem Lithium unter Argon-atmosphäre in 330 ml wasserfreiem Äther bereitetem Äthyllithium wird tropfenweise die Lösung von 11 g 2-Methoxy-2',4'-dihydroxy-benzophenon in 60 ml wasserfreiem Tetrahydrofuran gegeben, wobei darauf zu achten ist, daß die Temperatur nicht über −5°C ansteigt. Das Reaktionsgemisch wird 30 Minuten lang bei 0°C und dann 2 Stunden lang bei Raumtemperatur gerührt. Das Gemisch wird unter Kühlen in 10 %ige wäßrige Ammoniumchloridlösung gegossen, die organische Phase abgetrennt, und die wäßrige Phase mit Äther extrahiert. Die organischen Phasen werden vereinigt, mit Wasser neutral gewaschen, über wasserfreiem Magnesiumsulfat getrocknet und nach Klären mit Aktivkohle im Vakuum vom Lösungsmittel befreit. Der feste Rückstand wird aus einem n-Hexan/Äthylacetat-Gemisch umkristallisiert. Man erhält 8,3 g der Titelverbindung, die bei 141—142°C schmilzt.

Elementaranalyse für die Summenformel $C_{16}H_{18}O_4$
berechnet, %: C 70,05 H 6,61
gefunden, %: C 70,21 H 6,76

Durch entsprechende Wahl der Ausgangsverbindungen können auf die in den Beispielen beschriebene Weise die folgenden Verbindungen hergestellt werden.

4-[1-(4-Chlorphenyl)-1-hydroxy-propyl]-resorcin
Schmelzpunkt: 88°C

Elementaranalyse für die Summenformel $C_{15}H_{15}ClO_3$
berechnet, %: C 64,63 H 5,42 Cl 12,72
gefunden, %: C 64,42 H 5,54 Cl 12,88

4-[1-(4-Trifluormethyl-phenyl)-1-hydroxy-propyl]-resorcin
Elementaranalyse für die Summenformel $C_{16}H_{15}F_3O_3$
berechnet, %: C 61,53 H 4,84 F 18,25
gefunden, %: C 61,63 H 4,78 F 18

4-[1-(3-Chlorphenyl)-1-hydroxy-propyl]-resorcin
Schmelzpunkt: 144—145°C
Elementaranalyse für die Summenformel $C_{15}H_{15}ClO_3$
berechnet, %: C 64,63 H 5,42 Cl 12,72
gefunden, %: C 64,51 H 5,60 Cl 12,95

4-[1-(2,5-Dimethylphenyl)-1-hydroxy-propyl]-resorcin
Schmelzpunkt: 110°C
Elementaranalyse für $C_{17}H_{20}O_3$
berechnet, %: C 74,97 H 7,40
gefunden, %: C 75,10 H 7,33

4-[1-(4-Trifluormethyl-phenyl)-1-hydroxy-propyl]-resorcin
Schmelzpunkt: 141—142°C
Elementaranalyse für die Summenformel $C_{16}H_{15}F_3O_3$
berechnet, %: C 61,53 H 4,84 F 18,25
gefunden, %: C 61,58 H 4,90 F 18,21

Beispiel 5

Aus den erfindungsgemäßen Verbindungen können zum Beispiel die folgenden Arzneimittelpräparate hergestellt werden.

*Tabletten*

Zusammensetzung einer Tablette:

| | |
|---|---|
| Wirkstoff | 100,0 mg |
| Lactose | 184,0 mg |
| Kartoffelstärke | 80,0 mg |
| Polyvinylpyrrolidon | 8,0 mg |
| Talk | 12,0 mg |
| Magnesiumstearat | 2,0 mg |
| Aerosil® (kolloidales $SiO_2$) | 2,0 mg |
| Ultraamylopektin | 12,0 mg |

Durch Naßgranulieren und Pressen werden aus den angegebenen Stoffen Tabletten von 400 mg Gewicht hergestellt.
Wirkstoff: 4-[1-(3-Trifluormethyl-phenyl)-1-hydroxy-propyl]-resorcin

*Dragees*

Die auf die beschriebene Weise erhaltenen Tabletten werden in an sich bekannter Weise mit einem aus Zucker und Talk bestehenden Überzug versehen und dann mit einem Gemisch aus Bienenwachs und Carnaubawachs poliert.
Gewicht eines Dragees: 500,0 mg

*Kapseln*
Zusammensetzung der Füllung einer Kapsel:

| | |
|---|---|
| Wirkstoff | 50,0 mg |
| Lactose | 100,0 mg |
| Talk | 2,0 mg |
| Kartoffelstärke | 30,0 mg |
| mikrokristalline Cellulose | 8,0 mg |

Der Wirkstoff wird gründlich mit den Hilfsstoffen vermischt, das Gemisch durch ein Sieb der Maschenweite 0,32 mm gesiebt und dann in Hartgelatinekapseln (Größe 4) gefüllt.
Wirkstoff: 4-[1-(3-Trifluormethyl-phenyl)-1-hydroxy-propyl]-resorcin

*Suppositorien*
Zusammensetzung eines Suppositoriums:

| | |
|---|---|
| Wirkstoff | 100,0 mg |
| Lactose | 200,0 mg |
| Suppositorienmasse (z.B. Witepsol® H) | 1700,0 mg |

Die Grundmasse wird geschmolzen und die Schmelze auf 35°C gekühlt. Der Wirkstoff wird gründlich mit der Lactose vermischt und das Gemisch in einem Homogenisator mit der Schmelze homogenisiert. Das erhaltene Gemisch wird in gekühlte Suppositorienformen gegossen.
Gewicht eines Suppositoriums: 2000 mg
Wirkstoff: 4-[1-(3-Trifluormethyl-phenyl)-1-hydroxy-propyl]-resorcin

*Suspension*
100 ml Suspension enthalten:

| | |
|---|---|
| Wirkstoff | 1,0 g |
| Natriumhydroxyd | 0,26 g |
| Citronensäure | 0,30 g |
| Nipagin® (Natriumsalz des 4-Hydroxybenzoesäuremethylesters) | 0,10 g |
| Carbopol® 940 (Polyacrylsäure) | 0,30 g |
| Äthanol (96 %ig) | 1,00 g |
| Himbeeraroma | 0,60 g |
| Sorbit (70 %ige wäßrige Lösung) | 71,00 g |
| d'est. Wasser zum Auffüllen auf | 100,00 ml |

Das Nipagin® und die Citronensäure werden in 20 ml destilliertem Wasser gelöst. Zu der Lösung gibt man in kleinen Portionen, unter intensivem Rühren das Carbopol® und läßt die Lösung dann 10—12 Stunden stehen. Anschließend werden die mit 1 ml destilliertem Wasser bereitete Lösung des Natriumhydroxyds, dann die wäßrige Lösung des Sorbits und schließlich die äthanolische Lösung des Himbeeraromas zugegeben. Zu der auf diese Weise bereiteten Trägersubstanz gibt man in kleinen Portionen den Wirkstoff und homogenisiert des Ganze mit einem Mixer. Die Suspension wird mit destilliertem Wasser auf 100 ml aufgefüllt und in einer Kolloidmühle homogenisiert.
Wirkstoff: 4-[1-(3-Trifluormethyl-phenyl)-1-hydroxy-propyl]-resorcin

**Patentansprüche für die Vertragsstaaten: BE CH DE FR GB IT LI LU NL SE**

1. 4-[1-(Phenyl)-1-(hydroxy)-prop-1-yl]-resorcinderivate der allgemeinen Formel

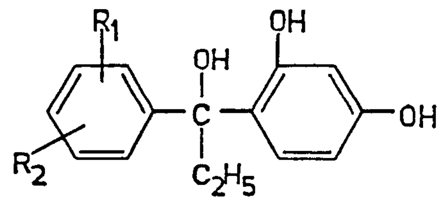

I ,

worin $R_1$ und $R_2$, die gleich oder verschieden sein können, für Wasserstoffatome, Halogenatome, Trihalogenmethylreste beziehungsweise Alkyl- beziehungsweise Alkoxyreste mit je 1 bis 3 Kohlenstoffatom(en) stehen.

2. 4-[1-(Phenyl)-1-(hydroxy)-prop-1-yl]-resorcinderivate nach Anspruch 1, dadurch gekennzeichnet, daß das beziehungsweise die Halogenatom(e), für das beziehungsweise die $R_1$ und/oder $R_2$ stehen kann beziehungsweise können, [ein] Chlor-, Fluor- und/oder Bromatom(e) ist beziehungsweise sind.

3. 4-[1-(Phenyl)-1-(hydroxy)-prop-1-yl]-resorcinderivate nach Anspruch 1 oder 2, dadurch gekennzeichnet, daß der beziehungsweise die Trihalogenmethylrest(e), für den beziehungsweise die $R_1$ und/oder $R_2$ stehen kann beziehungsweise können, [ein] Trifluormethylrest(e) und/oder Trichlormethylrest(e) ist beziehungsweise sind.

4. 4-[1-(Phenyl)-1-(hydroxy)-prop-1-yl]-resorcinderivate nach Anspruch 1 bis 3, dadurch gekennzeichnet, daß der beziehungsweise die Alkyl- und/oder Alkoxyrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

5. Die 4-[1-(Phenyl)-1-(hydroxy)-prop-1-yl]-resorcinderivate
4-[1-(3-Trifluormethyl-phenyl)-1-hydroxy-propyl]-resorcin,
4-[1-(2-Trifluormethyl-phenyl)-1-hydroxy-propyl]-resorcin,
4-[1-(4-Fluorphenyl)-1-hydroxy-propyl]-resorcin,
4-[1-(2-Methoxyphenyl)-1-hydroxy-propyl]-resorcin,
4-[1-(4-Chlorphenyl)-1-hydroxy-propyl]-resorcin,
4-[1-(4-Trifluormethyl-phenyl)-1-hydroxy-propyl]-resorcin,
4-[1-(3-Chlorphenyl)-1-hydroxy-propyl]-resorcin und
4-[1-(2,5-Dimethylphenyl)-1-hydroxy-propyl]-resorcin.

6. Verfahren zur Herstellung der Verbindungen nach Anspruch 1 bis 5, dadurch gekennzeichnet, daß man in an sich bekannter Weise

a) 2,4-Dihydroxypropiophenon mit eine Phenylgruppe aufweisenden Grignard-Verbindungen der allgemeinen Formel

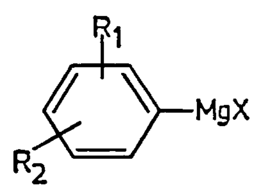

II ,

worin
$R_1$ und $R_2$ wie in den Ansprüchen 1 bis 4 festgelegt sind und
X für ein Halogenatom steht, umsetzt oder

b) 2,4-Dihydroxypropiophenon mit eine Phenylgruppe aufweisenden metallorganischen Verbindungen der allgemeinen Formel

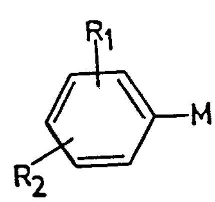

III ,

worin
$R_1$ und $R_2$ wie in den Ansprüchen 1 bis 4 festgelegt sind und

M für ein Alkalimetallatom, vorzugsweise Lithium-, Natriu- oder Kaliumatom, steht umsetzt oder
c) 2,4-Dihydroxybenzophenone der allgemeinen Formel

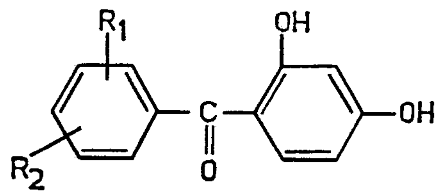

IV ,

worin $R_1$ und $R_2$ wie in den Ansprüchen 1 bis 4 festgelegt sind, mit eine Äthylgruppe aufweisenden metallorganischen Verbindungen, insbesondere Äthylmagnesiumhalogeniden oder Äthyllithium, umsetzt.

7. Arzneimittel, gekennzeichnet durch einen Gehalt an 1 oder mehr Verbindung(en) nach Anspruch 1 bis 5 als Wirkstoff(en), zweckmäßigerweise zusammen mit 1 oder mehr üblichen Konfectionierungsmittel(n).

**Patentansprüche für den Vertragsstaat: AT**

1. Verfahren zur Herstellung von 4-[1-(Phenyl)-1-(hydroxy)-prop-1-yl]-resorcinderivaten der allgemeinen Formel

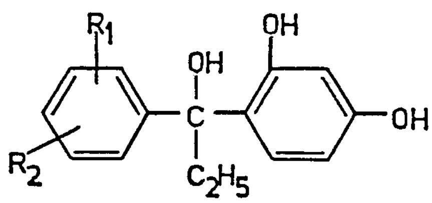

I ,

worin $R_1$ und $R_2$, die gleich oder verschieden sein können, für Wasserstoffatome, Halogenatome, Trihalogenmethylreste beziehungsweise Alkyl- beziehungsweise Alkoxyreste mit je 1 bis 3 Kohlenstoffatom(en) stehen, dadurch gekennzeichnet, daß man in an sich bekannter Weise

a) 2,4-Dihydroxypropiophenon mit eine Phenylgruppe aufweisenden Grignard-Verbindungen der allgemeinen Formel

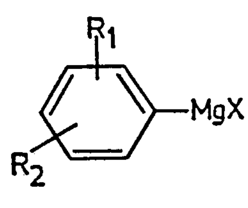

II ,

worin
$R_1$ und $R_2$ die oben angegebene Bedeutung haben und
X für ein Halogenatom steht, umsetzt oder
b) 2,4-Dihydroxypropiophenon mit eine Phenylgruppe aufweisenden metallorganischen Verbindungen der allgemeinen Formel

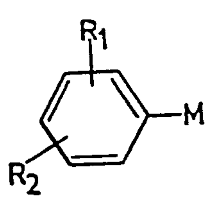

III ,

worin
$R_1$ und $R_2$ die oben angegebene Bedeutung haben und

M für ein Alkalimetallatom, vorzugsweise Lithium-, Natriu- oder Kaliumatom, steht umsetzt oder
c) 2,4-Dihydroxybenzophenone der allgemeinen Formel

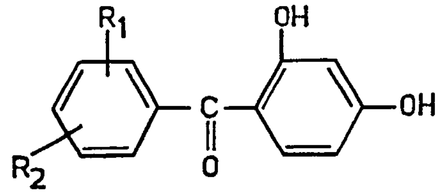

IV ,

worin $R_1$ und $R_2$ die oben angegebene Bedeutung haben, mit eine Äthylgruppe aufweisenden metallorganischen Verbindungen, insbesondere Äthylmagnesiumhalogeniden oder Äthyllithium, umsetzt.

2. Verfahren nach Anspruch 1, dadurch gekennzeichnet, daß solche 4-[1-(Phenyl)-1-(hydroxy)-prop-1-yl]-resorcinderivate hergestellt werden, bei denen das bzw. die Halogenatom(e), für das beziehungsweise die $R_1$ und/oder $R_2$ stehen kann beziehungsweise können, [ein] Chlor-, Fluor- und/oder Bromatom(e) ist beziehungsweise sind.

3. Verfahren nach Anspruch 1 oder 2, dadurch gekennzeichnet, daß solche 4-[1-(Phenyl)-1-(hydroxy)-prop-1-yl]-resorcinderivate hergestellt werden, bei denen der beziehungsweise die Trihalogenmethylrest(e), für den beziehungsweise die $R_1$ und/oder $R_2$ stehen kann beziehungsweise können, [ein] Trifluormethylrest(e) und/oder Trichlormethylrest(e) ist beziehungsweise sind.

4. Verfahren nach Anspruch 1 bis 3, dadurch gekennzeichnet, daß solche 4-[1-(Phenyl)-1-(hydroxy)-prop-1-yl]-resorcinderivate hergestellt werden, bei denen der beziehungsweise die Alkyl- und/oder Alkoxyrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

**Revendications pour les Etats contractants: BE CH DE FR GB IT LI LU NL SE**

1. Dérivés du 4-[1-(phényl)-1-(hydroxy)-prop-1-yl]-résorcinol de formule générale

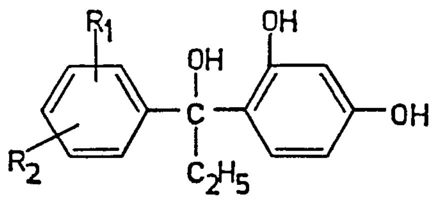

I ,

dans laquelle $R_1$ et $R_2$, qui peuvent être identiques ou différents, représentent des atomes d'hydrogène, des atomes d'halogène, des radicaux trihalogénométhyle ou des radicaux alcoyle ou alcoxy ayant chacun 1 à 3 atomes de carbone.

2. Dérivés du 4-[1-(phényl)-1-(hydroxy)-prop-1-yl]-résorcinol selon la revendication 1, caractérisés en ce que le ou les atomes d'halogène que peut ou peuvent respectivement représenter $R_1$ et/ou $R_2$, est ou sont respectivement un ou des atomes de chlore, de fluor et/ou de brome.

3. Dérivés du 4-[1-(phényl)-1-(hydroxy)-prop-1-yl]-résorcinol selon la revendication 1 ou 2, caractérisés en ce que le ou les radicaux trihalogénométhyle que peut ou peuvent respectivement représenter $R_1$ et/ou $R_2$, est ou sont respectivement un ou des radicaux trifluorométhyle et/ou un ou des radicaux trichlorométhyle.

4. Dérivés du 4-[1-(phényl)-1-(hydroxy)-prop-1-yl]-résorcinol selon les revendications 1 à 3, caractérisés en ce que le ou les radicaux alcoyle et/ou alcoxy que peut ou peuvent respectivement représenter $R_1$ et/ou $R_2$, est ou sont respectivement celui ou ceux ayant 1 ou 2, en particulier 1, atomes de carbone.

5. Les dérivés de 4-[1-(phényl)-1-(hydroxy)-prop-1-yl]-résorcinol
4-[1-(3-Trifluorométhyl-phényl)-1-hydroxy-propyl]-résorcinol,
4-[1-(2-Trifluorméthyl-phényl)-1-hydroxy-propyl]-résorcinol,
4-[1-(4-Fluorophényl)-1-hydroxy-propyl]-résorcinol,
4-[1-(2-Méthoxyphényl)-1-hydroxy-propyl]-résorcinol,
4-[1-(4-Chlorophényl)-1-hydroxy-propyl]-résorcinol,
4-[1-(4-Trifluorométhyl-phényl)-1-hydroxy-propyl]-résorcinol,
4-[1-(3-Chlorophényl)-1-hydroxy-propyl]-résorcinol et
4-[1-(2,5-Diméthylphényl)-1-hydroxy-propyl]-résorcinol.

6. Procédé pour la préparation des composés selon les revendications 1 à 5, caractérisé en ce que, d'une manière connue en soi

a) on fait réagir la 2,4-dihydroxypropiophénone avec des composés de Grignard comportant un groupe phényle, de formule générale

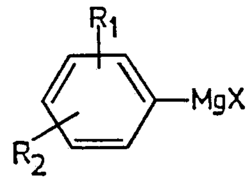

II ,

dans laquelle $R_1$ et $R_2$ sont définis comme spécifié dans les revendications 1 à 4, et X représente un atome d'halogène, ou

b) on fait réagir la 2,4-dihydroxypropiophénone avec des composés organométalliques comportant un groupe phényle, de formule générale

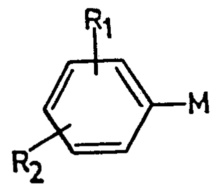

III ,

dans laquelle $R_1$ et $R_2$ sont définis comme spécifié dans les revendications 1 à 4, et M représente un atome de métal alcalin, de préférence un atome de lithium, de sodium ou de potassium, ou

c) on fait réagir les 2,4-dihydroxybenzophénones de formule générale

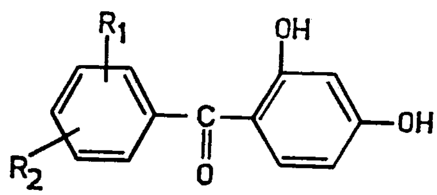

IV ,

dans laquelle $R_1$ et $R_2$ sont définis comme spécifié dans les revendications 1 à 4, avec des composés organométalliques comportant un groupe éthyle, en particulier les halogénures d'éthylmagnésium ou l'éthyllithium.

7. Médicaments, caractérisés par une teneur en un ou plusieurs composés selon les revendications 1 à 6 en tant que substance(s) active(s), d'une manière convenable conjointement avec un ou plusieurs agents de confection courants.

**Revendications pour l'Etat contractant: AT**

1. Procédé pour la préparation des dérivés du 4-[1-(phényl)-1-(hydroxy)-prop-1-yl]-résorcinol de formule générale

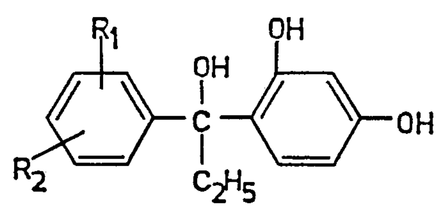

I ,

dans laquelle $R_1$ et $R_2$, qui peuvent être identiques ou différents, représentent des atomes d'hydrogène, des atomes d'halogène, des radicaux trihalogénométhyle ou des radicaux alcoyle ou alcoxy ayant chacun 1 à 3 atomes de carbone, caractérisé en ce que, d'une manière connue en soi

a) on fait réagir la 2,4-dihydroxypropiophénone avec des composés de Grignard comportant un groupe phényle, de formule générale

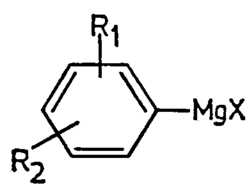

II ,

dans laquelle $R_1$ et $R_2$ sont définis comme spécifié ci-dessus et X représente un atome d'halogène, ou

b) on fait réagir la 2,4-dihydroxypropiophénone avec des composés organométalliques comportant un groupe phényle, de formule générale

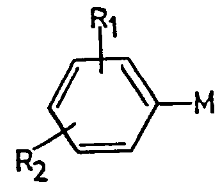

III ,

dans laquelle $R_1$ et $R_2$ sont définis comme spécifié ci-dessus et M représente un atome de métal alcalin, de préférence un atome de lithium, de sodium ou de potassium, ou

c) on fait réagir les 2,4-dihydroxybenzophénones de formule générale

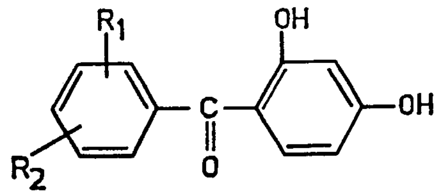

IV ,

dans laquelle $R_1$ et $R_2$ sont définis comme spécifié dans les revendications 1 à 4, avec des composés organométalliques comportant un groupe éthyle, en particulier les halogénures d'éthylmagnésium ou l'éthyllithium.

2. Procédé selon la revendication 1, caractérisé en ce que les dérivés du 4-[1-(phényl)-1-(hydroxy)-prop-1-yl]-résorcinol sont préparés, en ce que le ou les atomes d'halogène que peut ou peuvent respectivement représenter $R_1$ et/ou $R_2$, est ou sont respectivement un ou des atomes de chlore, de fluor et/ou de brome.

3. Procédé selon la revendication 1 ou 2, caractérisé en ce que les dérivés du 4-[1-(phényl)-1-(hydroxy)-prop-1-yl]-résorcinol sont préparés, en ce que le ou les radicaux trihalogénométhyle que peut ou peuvent respectivement représenter $R_1$ et/ou $R_2$, est ou sont respectivement ou ou des radicaux trifluorméthyle et/ou un ou des radicaux trichlorométhyle.

4. Procédé selon les revendications 1 à 3, caractérisé en ce que les dérivés du 4-[1-(phényl)-1-(hydroxy)-prop-1-yl]-résorcinol sont préparés, en ce que le ou les radicaux alcoyle et/ou alcoxy que peut ou peuvent respectivement représenter $R_1$ et/ou $R_2$, est ou sont respectivement celui ou ceux ayant 1 ou 2, en particulier 1, atomes de carbone.

**Claims for the Contracting States: BE CH DE FR GB IT LI LU NL SE**

1. 4-[1-(phenyl)-1(hydroxy)-prop-1-yl]-resorcin derivatives of the general formula

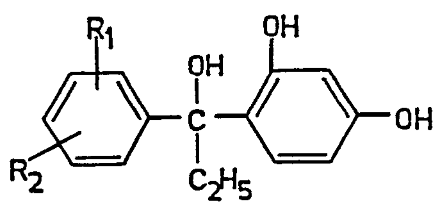

I ,

wherein $R_1$ and $R_2$ may be equal or different and stand for hydrogen atoms, halogen atoms, trihalomethyl residues and/or alkyl residues or alkoxy residues having 1 to 3 carbon atom(s).

2. 4-[1-(phenyl)-1(hydroxy)-prop-1-yl]-resorcin derivatives according to claim 1, characterized in that the halogen atom(s) for which $R_1$ and/or $R_2$ may stand consist(s) of (a) chlorine, fluorine and/or bromine atom(s).

3. 4-[1-(phenyl)-1(hydroxy)-prop-1-yl]-resorcin according to claim 1 or 2, characterized in that the trihalomethyl residue(s) for which $R_1$ and/or $R_2$ may stand consist(s) of (a) trifluoromethyl residue(s) and/or trichloromethyl residue(s).

4. 4-[1-(phenyl)-1(hydroxy)-prop-1-yl]-resorcin according to claims 1 to 3, characterized in that the alkyl residue(s) and/or alkoxy residue(s) for which $R_1$ and/or $R_2$ may stand consist(s) of such residue(s) which contain(s) 1 or 2 and particularly 1 carbon atom(s).

5. The 4-[1-(phenyl)-1(hydroxy)-prop-1-yl]-resorcin derivatives

4-[1-(3-trifluoromethyl-phenyl)-1-hydroxy-propyl]-resorcin,

4-[1-(2-trifluoromethyl-phenyl)-1-hydroxy-propyl]-resorcin,

4-[1-(4-fluorophenyl)-1-hydroxy-propyl]-resorcin,

4-[1-(2-methoxyphenyl)-1-hydroxy-propyl]-resorcin,

4-[1-(4-chlorophenyl)-1-hydroxy-propyl]-resorcin,
4-[1-(4-trifluoromethyl-phenyl)-1-hydroxy-propyl]-resorcin,
4-[1-(3-chlorophenyl)-1-hydroxy-propyl)]-resorcin and
4-[1-(2,5-dimethylphenyl)-1-hydroxy-propyl]-resorcin.

6. A process of producing the compounds according to claims 1 to 5, characterized in that, in a manner known per se,

a) 2,4-dihydroxypropiophenone is reacted with Grignard compounds which contain a phenyl group and have the general formula

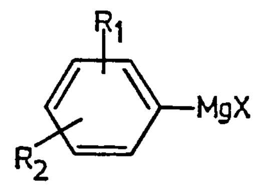

II ,

wherein

$R_1$ and $R_2$ are as defined hereinbefore and

X stands for a halogen atom or

b) 2,4-dihydroxypropiophenone is reacted with organometallic compounds which contain a phenyl group and have the general formula

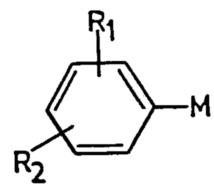

III ,

wherein

$R_1$ and $R_2$ are as defined hereinbefore and

M stands for an alkali metal atom, preferably a lithium, sodium or potassium atom or

c) 2,4-dihydroxybenzophenones of the general formula

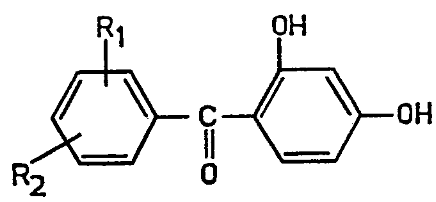

IV ,

wherein $R_1$ and $R_2$ are as defined hereinbefore are reacted with organometallic compounds which contain an ethyl group, particularly ethylmagnesium halides or ethyllithium.

7. Medicaments, characterized by a content of 1 or more compound(s) according to claims 1 to 5 as [an] active principle(s), suitably together with 1 or more usual processing agent(s).

**Claims for the Contracting State: AT**

1. A process for producing 4-[1-(phenyl)-1(hydroxy)-prop-1-yl]-resorcin derivatives of the general formula

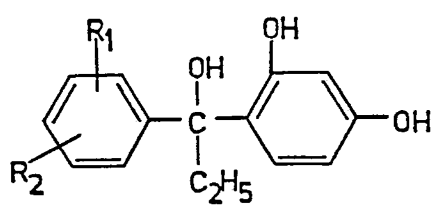

I ,

wherein $R_1$ and $R_2$ may be equal or different and stand for hydrogen atoms, halogen atoms, trihalomethyl residues and/or alkyl residues or alkoxy residues having 1 to 3 carbon atom(s), characterized in that, in a manner known per se,

a) 2,4-dihydroxypropiophenone is reacted with Grignard compounds which contain a phenyl group and have the general formula

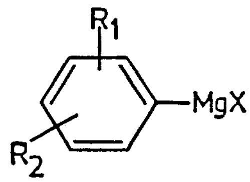

II ,

wherein

$R_1$ and $R_2$ are as defined hereinbefore and

X stands for a halogen atom or

b) 2,4-dihydroxypropiophenone is reacted with organometallic compounds which contain a phenyl group and have the general formula

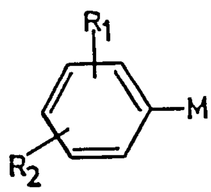

III ,

wherein

$R_1$ and $R_2$ are as defined hereinbefore and

M stands for an alkali metal atom, preferably a lithium, sodium or potassium atom or

c) 2,4-dihydroxybenzophenones of the general formula

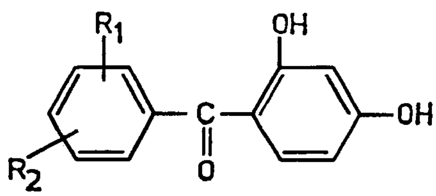

IV ,

wherein $R_1$ and $R_2$ are as defined hereinbefore are reacted with organometallic compounds which contain an ethyl group, particularly ethylmagnesium halides or ethyllithium.

2. A process according to claim 1, characterized in that 4-[1-(phenyl)-1(hydroxy)-prop-1-yl]-resorcin derivatives are prepared, wherein the halogen atom(s) for which $R_1$ and/or $R_2$ may stand consist(s) of (a) chlorine, fluorine and/or bromine atom(s).

3. A process according to claims 1 or 2, characterized in that 4-[1-(phenyl)-1(hydroxy)-prop-1-yl]-resorcin is prepared, wherein the trihalomethyl residue(s) for which $R_1$ and/or $R_2$ may stand consist(s) of (a) trifluoromethyl residue(s) and/or trichloromethyl residue(s).

4. A process according to claims 1 to 3, characterized in that 4-[1-(phenyl)-1(hydroxy)-prop-1-yl]-resorcin is prepared wherein the alkyl residue(s) and/or alkoxy residue(s) for which $R_1$ and/or $R_2$ may stand consist(s) of such residue(s) which contain(s) 1 or 2 and particularly 1 carbon atom(s).